(19) Europäisches Patentamt
European Patent Office
Office européen des brevets

(11) Veröffentlichungsnummer: **0 161 580**
**B1**

(12) **EUROPÄISCHE PATENTSCHRIFT**

(45) Veröffentlichungstag der Patentschrift: **07.11.90**

(21) Anmeldenummer: **85105310.8**

(22) Anmeldetag: **02.05.85**

(51) Int. Cl.$^5$: **C 07 D 491/048,**
C 07 D 233/32,
C 07 D 409/06, C 07 D 405/06
// (C07D491/048, 307:00,
235:00)

(54) **Verfahren zur Herstellung eines Lactons.**

(30) Priorität: **18.05.84 CH 2458/84**
**22.02.85 CH 825/85**

(43) Veröffentlichungstag der Anmeldung:
**21.11.85 Patentblatt 85/47**

(45) Bekanntmachung des Hinweises auf die Patenterteilung:
**07.11.90 Patentblatt 90/45**

(84) Benannte Vertragsstaaten:
**AT BE CH DE FR GB IT LI NL**

(56) Entgegenhaltungen:
**EP-A-0 084 892**

(73) Patentinhaber: **F. HOFFMANN-LA ROCHE AG**
**Postfach 3255**
**CH-4002 Basel (CH)**

(72) Erfinder: **Pauling, Horst, Dr.**
**Ruchholzstrasse 39**
**CH-4103 Bottmingen (CH)**
Erfinder: **Wehrli, Christof**
**Rheinstrasse 40**
**CH-4127 Birsfelden (CH)**

(74) Vertreter: **Cottong, Norbert A. et al**
**Grenzacherstrasse 124 Postfach 3255**
**CH-4002 Basel (CH)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 161 580 B1

**Beschreibung**

Die vorliegende Erfindung betrifft ein neues Verfahren zur Herstellung des optisch aktiven Lactons der Formel

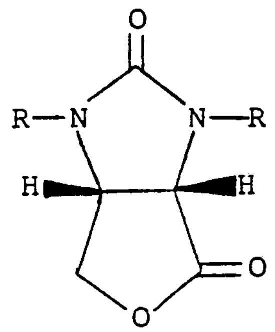

I

(3aS,6aR)

worin R den Benzylrest darstellt.

Dieses optisch aktive Lacton der Formel I ist ein bekanntes, wertvolles Zwischenprodukt in der Synthese von (+)-Biotin, sowie von Derivaten und verwandten Verbindungen hiervon.

Unter der Bezeichnung "(3aS, 6aR)", im Zusammenhang mit der Formel I, ist im Rahmen der vorliegenden Erfindung derjenige Antipode zu verstehen, welcher in Benzol oder Chloroform rechtsdrehend ist. Dieser Antipode wird im Nachfolgenden (+)-Lacton bezeichnet.

Aus der deutschen Patentschrift No. 2058 248 sowie aus der europäischen Patentpublikation No. 44 158 sind bereits Verfahren zur Herstellung des (+)-Lactons der Formel I bekannt. Im ersteren Fall werden racemische Halbester in ihre optischen Antipoden aufgetrennt und der gewünschte Antipode wird in das (+)-Lacton der Formel I übergeführt. Im zweiten Fall wird eine Dicarbonsäure oder das entsprechende Anhydrid mit einem bestimmten optisch aktiven Amin umgesetzt, wobei die (S)-Amidsäure im Ueberschuss gebildet wird. Diese kann anschliessend, nach Veresterung der Carboxylgruppe, mit Natriumborhydrid reduziert und zum (+)-Lacton der Formel I hydrolysiert werden. Beide Verfahren weisen Nachteile auf, indem einerseits eine Racematspaltung durchgeführt werden muss, mit Recyclisierung des unerwünschten Antipoden und andererseits, zwecks Erzielung einer guten Ausbeute, die sich ebenfalls bildende (R)-Amidsäure, sowie weitere Nebenprodukte ebenfalls recyclisiert werden müssen. Zudem muss im letzteren Fall, die nach der Amidbildung noch freie Carboxylgruppe verestert werden, um die nötige Reduktion zu ermöglichen.

Es bestand somit ein Bedürfnis nach einem Verfahren, gemäss welchem (+)-Lacton der Formel I in hoher Ausbeute, mit grosser optischer Reinheit, ohne notwendige Recyclisierung von unerwünschten Nebenprodukten bzw. ohne vorhergehende Amidbildung erhalten werden kann. Dies ist erfindungsgemäss nunmehr gelungen. Es wurde nämlich überraschend gefunden, dass durch Umsetzung des Cycloanhydrids der Formel

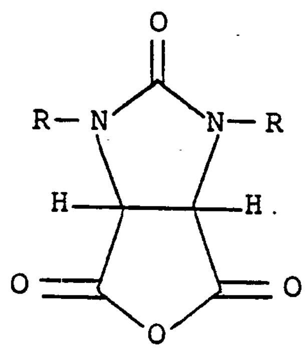

II

worin R die obige Bedeutung hat, mit bestimmten chiralen Alkoholen, praktisch ausschliesslich der gewünschte Halbester gebildet wird, welcher durch Reduktion leicht in das (+)-Lacton übergeführt werden kann.

Das erfindungsgemässe Verfahren ist demnach dadurch gekennzeichnet, dass man das Cycloanhydrid der Formel II mit einem sekundären, chiralen Alkohol der allgemeinen Formel

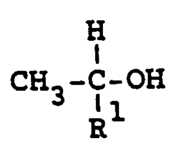

III

worin $R^1$ einen Rest der Formel

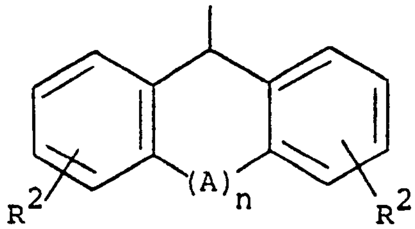

(a)

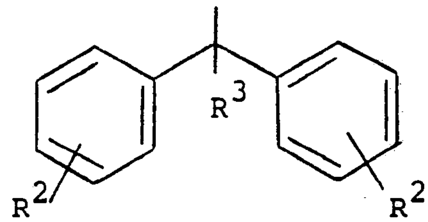

(b)

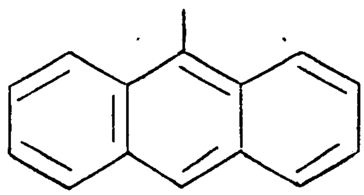

(c)

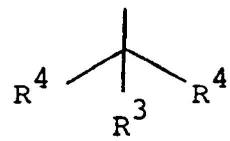

(d)

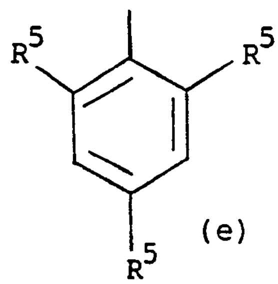

(e)

oder

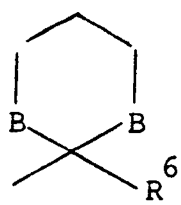

(f)

darstellt, worin $R^2$ Wasserstoff, Halogen, niederes Alkyl oder niederes Alkoxy, $R^3$ Wasserstoff oder Hydroxy, oder falls $R^2$ in dem Rest (b) Wasserstoff ist auch niederes Alkyl, niederes Alkoxy oder Phenyl, $R^4$ Cycloalkyl, gegebenenfalls Chlor oder Methyl substituiertes Phenyl, Thienyl oder 2-Furyl, $R^5$ Wasserstoff oder niederes Alkyl, $R^6$ niederes Alkyl oder Phenyl, A Schwefel oder eine Methylengruppe, B Schwefel, $-SO_2-$ oder eine Methylengruppe bedeuten und n die Zahl 1 ist falls A Schwefel darstellt oder die Zahl 1 oder 2 ist falls A eine Methylengruppe bedeutet, umsetzt und den erhaltenen Halbester der allgemeinen Formel

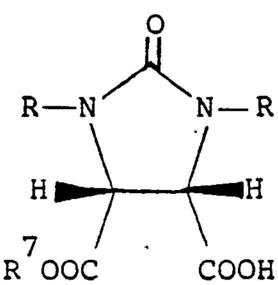

IV

worin $R^7$ den Rest

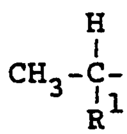

darstellt und R und $R^1$ die obige Bedeutung haben, mit einem komplexen Borhydrid reduziert.

Der Ausdruck "niederes Alkyl" bedeutet im Rahmen der vorliegenden Erfindung geradkettige oder verzweigte Alkylgruppen mit 1 bis 5 Koholenstoffatomen, wie Methyl, Aethyl, Propyl, Isopropyl, n-Butyl, Isobutyl, Pentyl und dergleichen. Der Ausdruck "niederes Alkoxy" bedeutet Gruppen in denen der Alkylrest die vorhergehende Bedeutung hat. Der Ausdruck "Halogen" bedeutet Fluor, Chlor oder Brom. Der

Ausdruck Cycloalkyl bedeutet Gruppen mit 3 bis 7 Kohlenstoffatomen, wie Cyclopropyl, Cyclobutyl, Cyclopentyl, Cyclohexyl und Cycloheptyl.

Unter "komplexen Borhydriden" sind im Rahmen der vorliegenden Erfindung insbesondere solche zu verstehen, worin das Kation ein Alkalimetall, wie Lithium, Natrium oder Kalium, oder ein Tetraalkylammoniumion, wie Tetrabutylammonium, sein kann. Besonders bevorzugt ist Lithiumborhydrid.

Die verwendeten chiralen Alkohole der Formel III sind zum Teil bekannte und zum Teil neue Verbindungen und können in an sich bekannter Weise hergestellt werden. Hierbei muss jedoch darauf geachtet werden, dass die erhaltenen chiralen Alkohole jeweils enantiomer einheitlich sind. Bevorzugte Alkohole der Formel III sind diejenigen, worin $R^1$ einen Rest der Formel (d) darstellt.

Die Umsetzung des Cycloanhydrids der Formel II mit einem chiralen, sekundären Alkohol der Formel III kann in an sich bekannter Weise erfolgen. Zweckmässig geschieht dies unter Inertgas, wie beispielsweise Kohlendioxid, Argon, Stickstoff und dergleichen, und in einem unter den Reaktionsbedingungen inerten, wasserfreien, organischen Lösungsmittel. Als Lösungsmittel können insbesondere genannt werden aromatische Kohlenwasserstoffe wie Benzol, Toluol, Xylol, Anisol, Chlorbenzol und dergleichen, Aether wie Diäthyläther, Tetrahydrofuran, Dioxan oder Polyäther wie Monoglyme oder Diglyme und dergleichen, halogenierte Kohlenwasserstoffe wie Methylenchlorid, Chloroform oder auch Dimethylformamid, Dimethylsulfoxid, Acetonitril, Cyclohexen, Schwefelkohlenstoff usw. Bevorzugte Lösungsmittel sind hierbei aromatische Kohlenwasserstoffe und insbesondere die vorhergehend erwähnten.

Die Umsetzung kann, bevorzugt, in Gegenwart eines Katalysators durchgeführt werden. Als solche kommen hier in Frage beispielsweise tert. Amine wie etwa Diazabicyclooctan, Diazabicycloundecen, p-Dimethylaminopyridin und dergleichen oder auch Trialkylamine mit niederen Alkylresten, wie Triäthylamin usw. Falls ein Katalysator verwendet wird, erfolgt dies zweckmässig in stöchiometrischen Mengen. Weiterhin kann die Umsetzung bei einer Temperatur von etwa −70°C bis zur Rückflusstemperatur des Reaktionsgemisches erfolgen. Bevorzugt erfolgt die Umsetzung bei einer Temperatur von etwa −50°C bis etwa Raumtemperatur und insbesondere von etwa −30°C bis etwa 0°C. Der Druck ist bei dieser Reaktion keine kritische Grösse.

Eine besonders bevorzugte Ausführungsform des erfindungsgemässen Verfahrens besteht darin, dass man das Cycloanhydrid der Formel II mit einem [S]-1,1-Diarylpropanol oder [S]-1,1-Diarylpropan-1,2-diol, vorzugsweise mit [S]-1,1-Diphenylpropanol oder [S]-1,1-Diphenyl-1,2-propandiol, in einem aromatischen Kohlenwasserstoff, insbesondere Toluol, bei einer Temperatur von −20°C bis 0°C in Gegenwart stöchiometrischer Mengen Katalysator, insbesondere Diazabicyclooctan, durchführt.

Die Reduktion der Halbester der Formel IV kann in situ erfolgen oder auch nach deren Isolierung. Vor Durchführung der Reduktion wird die freie Carboxylgruppe zweckmässig in ein Salz übergeführt, sofern die Umsetzung des Cycloanhydrids der Formel II mit einem Alkohol der Formel III nicht bereits in Gegenwart eines Katalysators durchgeführt wurde. Die Reduktion selbst kann mittels eines komplexen Borhydrids und vorzugsweise mittels Lithiumborhydrid erfolgen. Die Reduktion erfolgt zweckmässig in einer Inertgasatmosphäre, beispielsweise unter Stickstoff oder Argon, in einem inerten organischen Lösungsmittel wie einem Aether, beispielsweise Dioxan oder Tetrahydrofuran oder einem Aether des Glycols oder Diäthylenglycols, beispielsweise Diäthylenglycoldimethyläther und bei einer Temperatur von etwa Raumtemperatur bis zur Rückflusstemperatur des Reaktionsgemisches. Das hierbei bevorzugt verwendete Lithiumborhydrid kann als solches eingesetzt werden oder in situ aus Natrium- oder Kaliumborhydrid und Lithiumchlorid oder Lithiumbromid gebildet werden.

Die folgenden Beispiele dienen zur Erläuterung der Erfindung und stellen keinerlei Einschränkung hiervorn dar.

Die in den Beispielen angegebene optische Reinheit "e.e." ist bezogen auf ein Lacton der Formel I mit $[\alpha]_D^{20} = +62,0°$ (1% $CHCl_3$).

## Beispiel 1

A) In einer unter Argon stehenden Apparatur werden 1,01 g (3 mMol) cis - 1,3 - Dibenzyl - hexahydro - 1H - furo[3,4-d]imidazol - 2,4,6 - trion in 10 ml Toluol und 0,637 g (3 mMol) [S]-1,1-Diphenyl-2-propanol ($[\alpha]_{365}^{20} = -218,6°$ (0,5 Aethanol)), vorgelegt. Zu dieser Suspension wird bei −10°C innert 30 Minuten eine Lösung von 0,168 g (1,5 mMol) Diazabicyclooctan in 10 ml Toluol zugetropft. Nach 18 Stunden bei −10°C wird die nun klare Lösung noch 1 Stunde bei Raumtemperatur gerührt. Dann wird mit 30 ml 0,1N HCl angesäuert und in 3 Scheidetrichter mit je 100 ml Aether extrahiert. Die organischen Phasen werden dreimal mit je 30 ml Wasser gewaschen, vereinigt, über Natriumsulfat getrocknet und eingeengt. Man erhält 2,8 g 5 - [−[S] - 1,1 - Diphenyl - 2 - propyl] - 4 - hydrogen - cis - 1,3 - dibenzyl - 2 - oxo - 4,5 - imidazolidin - dicarboxylat.

B) In einer unter Argon stehenden Apparatur werden 5,7 ml einer 1,05 molaren Lithiumborhydridlösung in Tetrahydrofuran (6 mMol) vorgelegt. Hierzu wird innert 1 Stunde bei 40°—45°C eine Lösung von 2,8 g (3 mMol) 5 - [−[S] - 1,1 - Diphenyl - 2 - propyl] - 4 - hydrogen - cis - 1,3 - dibenzyl - 2 - oxo - 4,5 - imidazolidin - dicarboxylat (hergestellt gemäss A)) in 20 ml Tetrahydrofuran und 0,42 ml Triäthylamin (3 mMol) zugetropft. Das Gemisch wird noch 2 Stunden bei 40°C gerührt und anschliessend mit 3,5 ml 3N HCl versetzt. Hierauf wird die Reaktionsmischung 30 Minuten bei 70°C gerührt und mit je 150 ml Aether in 2 Scheidetricher extrahiert. Die organischen Phasen werden dreimal mit je 50 ml Wasser gewaschen,

vereinigt, über Natriumsulfat getrocknet und eingeengt. Der Rückstand (1,65 g) wird an 150 g Kieselgel mit 600 ml n-Hexan/Aether (4:6, v/v) und anschliessend mit 800 ml Toluol/Aceton/Eisessig (92:5:3, v/v) chromatographiert. Man erhält 0,772 g (80%) (3aS,6aR) - 1,3 - Dibenzyl - dihydro - 1H - furo[3,4-d]-imidazol - 2,4(3H,3aH) - dion. $[\alpha]_D^{20} = +59,4°$ (1% $CHCl_3$), was einer optischen Reinheit von 95,8% e.e. entspricht. Nach Umkristallisation aus 7,7 ml Isopropanol hat das Produkt eine Drehung von $[\alpha]_D^{20} = +61,3°$ (1% in $CHCl_3$), was einer optischen Reinheit von 98,7% e.e. entspricht.

Beispiel 2

A) In einer unter Argon stehenden Apparatur werden 0,036 g (1 mMol) cis - 1,3 - Dibenzyl - hexahydro - 1H - furo[3,4-d]imidazol - 2,4,6 - trion, 5 ml Tetrahydrofuran und 0,238 (1 mMol) [+] - 10,11 - Dihydro - α - methyl - 5H - dibenzo[a,d] - cycloheptan - 5 - methanol ($[\alpha]_{365}^{20} = +326,8°$ (1% in Aethanol)) vorgelegt. Dazu wird innert 30 Minuten bei Raumtemperatur eine Lösung von 0,14 ml Triäthylamin in 5 ml Tetrahydrofuran zugetropft. Die Suspension löst sich während dem Zutropfen auf. Nach 18 Stunden werden 10 ml 1N HCl zugegeben und das Produkt zweimal mit je 100 ml Aether extrahiert. Die organischen Phasen werden dreimal mit je 20 ml Wasser gewaschen, vereinigt, über Natirumsulfat getrocknet und eingeengt. Man erhält 0,50 g (87%) 5 - [1 - (10,11 - Dihydro - 5H - dibenzo[a,d]cyclohepten - 5 - yl)äthyl] - 4 - hydrogen - cis - 1,3 - dibenzyl - 2 - oxo - 4,5 - imidazolidin - dicarboxylat.

B) In einer unter Argon stehenden Apparatur werden 5 ml einer 0,4 molaren Lithiumborhydridlösung in Tetrahydrofuran (2 mMol) vorgelegt. Zu dieser Lösung wird bei 40°C innert 30 Minuten eine Lösung von 0,5 g (0,87 mMol) 5 - [1 - (10,11 - Dihydro - 5H - dibenzo[a,d]cycloheptin - 5 - yl)äthyl] - 4 - hydrogen - cis - 1,3 - dibenzyl - 2 - oxo - 4,5 - imidazolidin - dicarboxylat (hergestellt gemäss A), 10 ml Tetrahydrofuran und 0,12 ml (0,87 mMol) Triäthylamin zugetropft. Das Gemsich wird noch 2,5 Stunden bei 40°C gerührt. Die Lösung wird auf 10°C gekühlt und dann mit 5 ml 3N HCl zersetzt. Anschliessend wird 30 Minuten bei 60°C gerührt und das Produkt zweimal mit je 100 ml Aether extrahiert. Die organischen Phasen werden dreimal mit je 20 ml Wasser gewaschen, vereinigt, über Natriumsulfat getrocknet und eingeengt. Die erhaltenen 0,60 g Rohprodukt werden an 150 g Kieselgel chromatographiert. Der chirale Akohol wird mit 600 ml Cyclohexan/Aether (6:4, v/v) eluiert, danach wird das erhaltene (+)-Lacton mit 800 ml eines Gemisches Toluol/Aceton/Eisessig (92:5:3, v/v) eluiert. Man erhält 0,229 g (82%) (3aS,6aR) - 1,3 - Dibenzyl - dihydro - 1H - furo[3,4-d]imidazol - 2,4(3H,3aH) - dion. $[\alpha]_D^{20} = +52.7°$ (1% $CHCl_3$), was einer optischen Reinheit von 85% e.e. entspricht.

Nach Umkristallisation von 0.2 g aus 2 ml Isopropanol hat das Produkt eine Drehung von $[\alpha]_D^{20} = +59°$ (1%, $CHCl_3$), was einer optischen Reinheit von 95% e.e. entspricht.

Beispiel 3

In einer unter Argon stehenden Apparatus werden 0,53 g (1,58 mMol) cis - 1,3 - Dibenzyl - hexhydro - 1H - furo[3,4-d]imidazol - 2,4,6 - trion, 0,354 g (1,58 mMol) (+) - 9,10 - Dihydro - α - methyl - 9 - anthracene - methanol $[\alpha]_{365}^{20} = +63,4°$ (1% Aethanol)), sowie 5 ml Tetrahydrofuran vorgelegt und dazu innert 30 Minuten bei Raumtemperatur eine Lösung von 0,22 ml (1,58 mMol) Triäthylamin in 5 ml Tetrahydrofuran zugetropft. Nach 18 Stunden wird die klare Lösung bei 40°C innert 30 Minuten zu 3 ml einer 1 molaren Lithiumborhydridlösung in Tetrahydrofuran (3 mMol) zugetropft. Nach 2 Stunden Rühren bei 40°C wird auf 10°C abgekühlt und mit 5 ml 3N HCl zersetzt. Nach 24 Stunden Rühren bei Raumtemperatur wird das Produkt zweimal mit je 10 ml Aether extrahiert und dreimal mit je 20 ml Wasser gewaschen. Die organischen Phasen werden vereinigt, über $Na_2SO_4$ getrocknet und eingeengt. Der Rückstand wird an 150 g Kielselgel mit 600 ml Cyclohexan/Aether (6:4, v/v) und anschliessend mit 800 ml Toluol/Aceton/Eisessig (92:6:3, v/v) chromatographiert. Man erhält 0,31 g des eingesetzten Alkohols mit $[\alpha]_{365}^{20} = +60,8°$ (1% Aethanol), und 0,404 g (3aS,6aR) - 1,3 - Dibenzyldihydro - 1H - furo[3,4-d]imidazol - 2,4(3H,3aH) - dion. $[\alpha]_D^{20} = +51,6°$ (1% $CHCl_3$), was einer optischen Reinheit von 83,2% e.e. entspricht.

Nach Umkristallisation von 0.3 g aus 3 ml Isopropanol hat das Produkt eine Drehung von $[\alpha]_D^{20} = +58,4°$ (1% in $CHCl_3$), was einer optischen Reinheit von 94,2% e.e. entspricht.

Beispiel 4

In einer unter Argon stehenden Apparatus werden 0,336 g (1 mMol) cis - 1,3 - Dibenzyl - hexahydro - 1H - furo[3,4-d]imidazol - 2,4,6 - trion in 3,3 ml Tetrahydrofuran vorgelegt. Dann wird innert 15 Minuten bei Raumtemperatur eine Lösung von 0,15 ml (1,1 mMol) Triäthylamin und 0,224 g (1 mMol) [S] - 1,1 - di - (3 - Thienyl) - 2 - propanol ($[\alpha]_{365}^{20} = -77,4°$ (1% in Aethanol)) in 3,3 ml Tetrahydrofuran zugetropft. Die Lösung wird dann 1,5 Stunden bei Raumptemperatur gerührt. Zu dieser Lösung werden bei 40°—45°C, unter Argonbegasung, 1,9 ml einer 1,04 molaren Lithiumborhydridlösung in Tetrahydrofuran (2 mMol) gegeben und 2 Stunden bei 40°—45°C gerührt. Nach dem Abkühlen werden bei 10°—15°C 3 ml 3N HCl zugetropft. Die erhaltene Mischung wird dann 30 Minuten bei 70°C gerührt und anschliessend mit je 100 ml Aether in 2 Scheidetrichter extrahiert. Die organischen Phasen werden viermal mit je 50 ml Wasser gewaschen, vereinigt, über Natriumsulfat getrocknet und eingeengt. Der Rückstand wird an 150 g Kieselgel mit 600 ml n-Hexan/Aether (4:6, v/v) und 800 ml Toluol/Aceton/Eisessig (92:5:3, v/v) chromatographiert. Man erhält (3aS,6aR) - 1,3 - Dibenzyl - dihydro - 1H - furo[3,4-d]imidazol - 2,4(3H,3aH) - dion mit $[\alpha]_D^{20} = +49,3°$ (1% $CHCl_3$), was einer optischen Reinheit von 79,5% e.e. entspricht.

Beispiel 5

In einer unter Argon stehenden Apparatur werden 0,673 g (2 mMol) cis - 1,3 - Dibenzyl - hexahydro - 1H - furo[3,4]imidazol - 2,4,6 - trion und 0,435 g (2 mMol) (−) - 3,3 - Diphenyl - 2 - butanol ($[\alpha]^{20}_{365}$ = −338,9° (1% Aethanol)) in 5 ml Toluol während 6 Stunden unter Rückfluss erhitzt. Nach dem Abkühlen werden 0.28 ml (2 mMol) Triäthylamin und 5 ml Tetrahydrofuran zugegeben. Die erhaltene Lösung wird innert 30 Minuten bei 40°C unter Argon zu einer Lösung von 4,8 ml einer 1 molaren Lithiumborhydridlösung in Tetrahydrofuran (4,8 mMol) zugetropft und 14 Stunden bei 40°C reagieren gelassen. Bei 10°C wird der Hydridüberschuss mit 5 ml 3N HCl zersetzt und 30 Minuten bei 60°C gerührt. Das erhaltene Produkt wird gemäss Beispiel 2 isoliert und gereinigt und man erhält 0,333 g (3aS,6aR) - 1,3 - Dibenzyl - dihydro - 1H - furo[3,4-d]imidazol - 2,4(3H,3aH) - dion. $[\alpha]^{20}_{D}$ = +38,7° (1% in $CHCl_3$), was einer optischen Reinheit von 62,4% e.e. entspricht.

Beispiel 6

In zu den Beispielen 1—5 analoger Weise wurde cis - 1,3 - Dibenzyl - hexahydro - 1H - furo[3,4-d]imidazol - 2,4,6 - trion mit verschiedenen Alkoholen umgesetzt und die Halbester reduziert. Die Ergebnisse sind in der folgenden Tabelle zusammengefasst.

Tabelle

| Alkohol | $[\alpha]^{20}_{365}$ (1% Aethanol) | Lacton der Formel I $[\alpha]^{20}_{D}$ (1% $CHCl_3$) | % e.e. |
|---|---|---|---|
| A | +5,0° | +38,0° | 61,3 |
| B | -55,7° ** | +45,0° | 72,6 |
| | | +51,8° * | 83,5 * |
| C | -1,7° ** | +45,6° | 73,6 |
| D | -186,8° | +45,9° | 74,0 |
| | | +54,5° * | 87,9 * |
| E | -151,1° | +46,0° | 74,2 |
| | | +49,1° * | 79,2 * |
| F | +29,1° ** | +51,7° | 83,4 |
| G | -212,9° | +52,3° | 84,4 |
| | | +55,0° * | 88,7 * |
| H | -338,9° | +53,6° | 86,5 |
| | | +59.0° * | 95.2 * |

* Lacton umkristallisiert aus Isopropanol

** $[\alpha]^{20}_{D}$ statt $[\alpha]^{20}_{365}$

A = (+)-α-Methyl-1-phenylcyclohexanmethanol

B = [S](-)-1-(2,4,6-Trimethylphenyl)-äthanol

C = (-)-1-(9-Anthryl)-äthanol

D = (-)-1-(2,4,6-Triäthylphenyl)-äthanol

E = (-)-1-(2,4,6-Triisopropylphenyl)-äthanol

F = [S]-(+)-1,1,1-Triphenyl-2-propanol

G = [S]-(-)-1,1-Diphenyl-2-propanol

H = (-)-3,3-Diphenyl-2-butanol

**Patentansprüche für die Vertragsstaaten: BE, CH, DE, FR, GB, IT, LI, NL**

1. Verfahren zur Herstellung des optisch aktiven Lactons der Formel

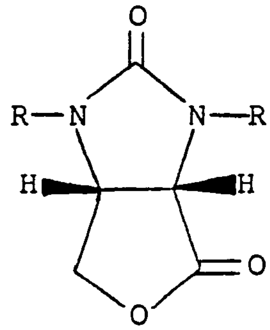

(3aS,6aR) I

worin R den Benzylrest darstellt, dadurch gekennzeichnet, dass man das Cycloanhydrid der Formel

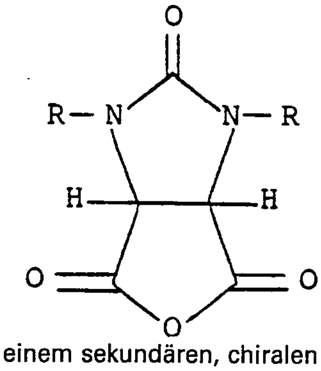

II

worin R obige Bedeutung hat, mit einem sekundären, chiralen Alkohol der allgemeinen Formel

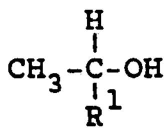

III

worin $R^1$ einen Rest der Formel

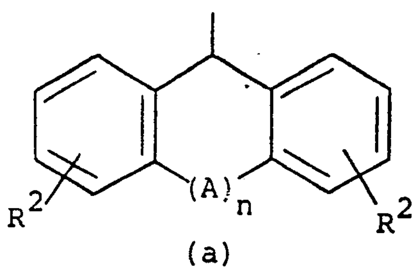

(a)

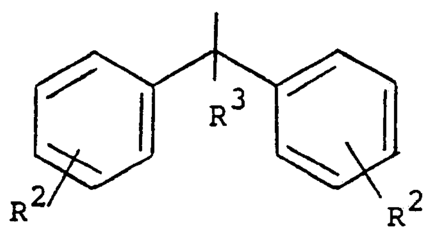

(b)

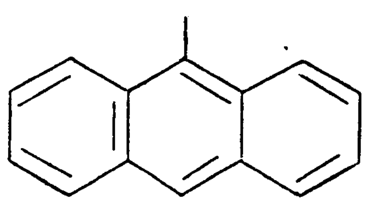

(c)

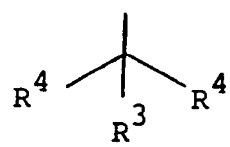

(d)

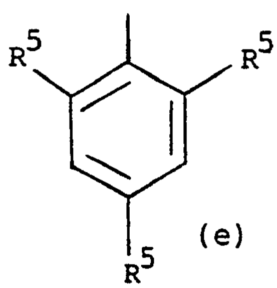

(e)

oder

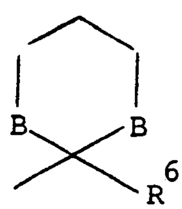

(f)

darstellt, worin $R^2$ Wasserstoff, Halogen, niederes Alkyl oder niederes Alkoxy, $R^3$ Wasserstoff oder Hydroxy, oder falls $R^2$ in dem Rest (b) Wasserstoff ist auch niederes Alkyl, niederes Alkoxy oder Phenyl, $R^4$ Cycloalkyl, gegebenenfalls Chlor oder Methyl substituiertes Phenyl, Thienyl oder 2-Furyl, $R^5$ Wasserstoff oder niederes Alkyl, $R^6$ niederes Alkyl oder Phenyl, A Schwefel oder eine Methylengruppe, B Schwefel, $-SO_2-$ oder eine Methylengruppe bedeuten und n die Zahl 1 ist falls A Schwefel darstellt oder die Zahl 1 oder 2 ist falls A eine Methylengruppe bedeutet, umsetzt und den erhaltenen Halbester der allgemeinen Formel

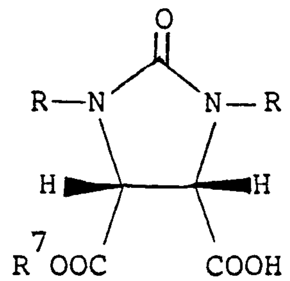

IV

worin $R^7$ den Rest

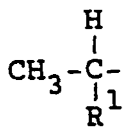

darstellt und R und $R^1$ die obige Bedeutung haben, mit einem komplexen Borhydrid reduziert.

2. Verfahren gemäss Anspruch 1, dadurch gekennzeichnet, dass man die Umsetzung des Cycloanhydrides der Formel II mit einem Alkohol der Formel III in Gegenwart eines tert. Amins als Katalysator, beispielsweise in Gegenwart von Triäthylamin, durchführt.

3. Verfahren gemäss Anspruch 2, dadurch gekennzeichnet, dass man den Katalysator in stöchiometrischen Mengen verwendet.

4. Verfahren gemäss einem der Ansprüche 1—3, dadurch gekennzeichnet, dass man die Reduktion eines Halbesters der Formel IV mit Lithiumborhydrid durchführt.

5. Verfahren gemäss einem der Ansprüche 1—4, dadurch gekennzeichnet, dass man sowohl die Umsetzung des Cycloanhydrids der Formel II mit einem Alkohol der Formel III als auch die Reduktion eines Halbesters der Formel IV unter Inertgas durchführt.

6. Verfahren gemäss einem der Ansprüche 1—5, dadurch gekennzeichnet, dass man die Umsetzung des Cycloanhydrids der Formel II mit einem Alkohol der Formel III bei einer Temperatur von etwa −50°C bis etwa Raumtemperatur durchführt.

7. Verfahren gemäss einem der Ansprüche 1—6, dadurch gekennzeichnet, dass man einen Alkohol der Formel III verwendet, worin $R^1$ einen Rest der Formel (d) darstellt.

8. Verfahren gemäss Anspruch 7, dadurch gekennzeichnet, dass man als Alkohol der Formel III ein [S]-1,1-Diarylpropanol oder ein [S]-1,1-Diarylpropan-1,2-diol verwendet.

9. Verfahren gemäss einem der Ansprüche 1—8, dadurch gekennzeichnet, dass man das Cycloanhydrid der Formel II mit [S]-1,1-Diphenylpropanol oder [S]-1,1-Diphenyl-1,2-propandiol in einem aromatischen Kohlenwasserstoff bei einer Temperatur von etwa −20°C bis etwa 0°C in Gegenwart von Diazabicyclooctan durchführt.

10. Optisch aktive Halbester der allgemeinen Formel

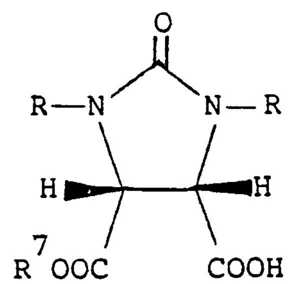

IV

worin R den Benzylrest und $R^7$ den Rest

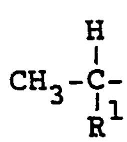

darstellt und $R^1$ einen Rest der Formel

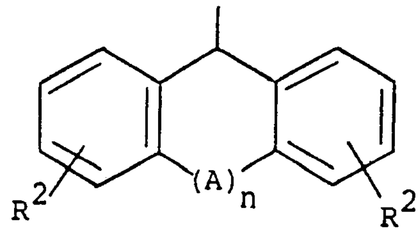

(a)

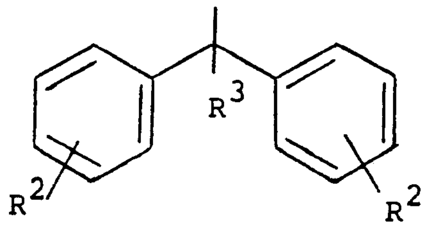

(b)

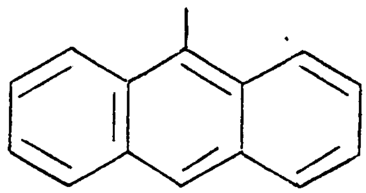

(c)

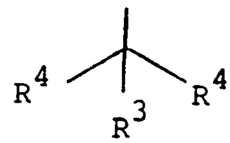

(d)

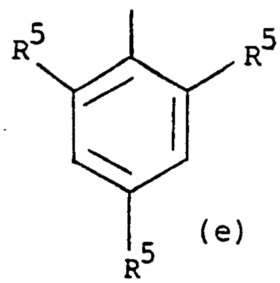

(e)

oder

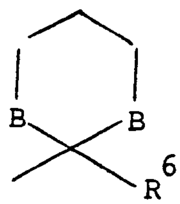

(f)

darstellt, worin $R^2$ Wasserstoff, Halogen, niederes Alkyl oder niederes Alkoxy, $R^3$ Wasserstoff oder Hydroxy, oder falls $R^2$ in dem Rest (b) Wasserstoff ist auch niederes Alkyl, niederes Alkoxy oder Phenyl, $R^4$ Cycloalkyl, gegebenenfalls Chlor oder Methyl substituiertes Phenyl, Thienyl oder 2-Furyl, $R^5$ Wasserstoff oder niederes Alkyl, $R^6$ niederes Alkyl oder Phenyl, A Schwefel oder eine Methylengruppe, B Schwefel, $-SO_2-$ oder eine Methylengruppe bedeuten und n die Zahl 1 ist falls A Schwefel darstellt oder die Zahl 1 oder 2 ist falls A eine Methylengruppe bedeutet.

**Patentansprüche für den Vertragsstaat: AT**

1. Verfahren zur Herstellung des optisch aktiven Lactons der Formel

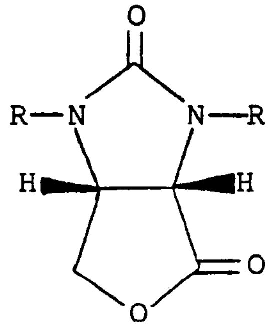

(3aS,6aR) I

worin R den Benzylrest darstellt, dadurch gekennzeichnet, dass man das Cycloanhydrid der Formel

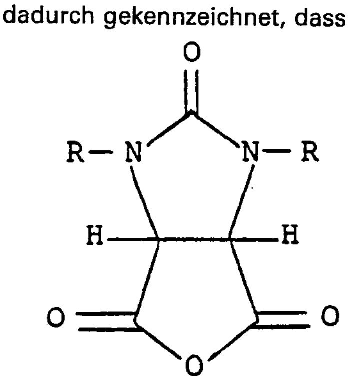

II

worin R obige Bedeutung hat, mit einem sekundären, chiralen Alkohol der allgemeinen Formel

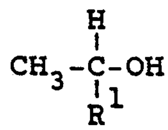

III

worin $R^1$ einen Rest der Formel

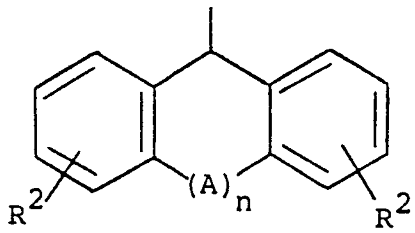

(a)

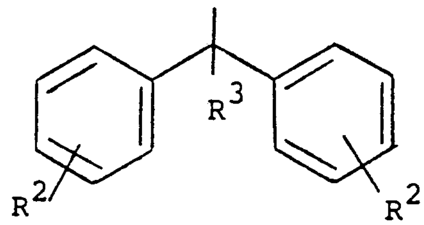

(b)

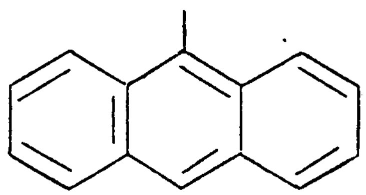

(c)

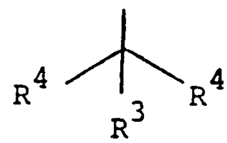

(d)

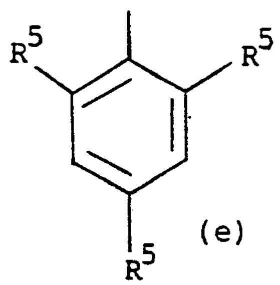

(e)

oder

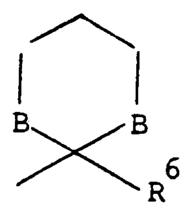

(f)

darstellt, worin $R^2$ Wasserstoff, Halogen, niederes Alkyl oder niederes Alkoxy, $R^3$ Wasserstoff oder Hydroxy, oder falls $R^2$ in dem Rest (b) Wasserstoff ist auch niederes Alkyl, niederes Alkoxy oder Phenyl, $R^4$ Cycloalkyl, gegebenenfalls Chlor oder Methyl substituiertes Phenyl, Thienyl oder 2-Furyl, $R^5$ Wasserstoff oder niederes Alkyl, $R^6$ niederes Alkyl oder Phenyl, A Schwefel oder eine Methylengruppe, B Schwefel, $-SO_2-$ oder eine Methylengruppe bedeuten und n die Zahl 1 ist falls A Schwefel darstellt oder die Zahl 1 oder 2 ist falls A eine Methylengruppe bedeutet, umsetzt und den erhaltenen Halbester der allgemeinen Formel

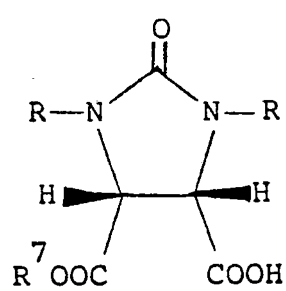

IV

worin $R^7$ den Rest

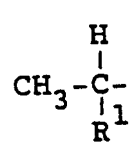

darstellt und R und $R^1$ die obige Bedeutung haben, mit einem komplexen Borhydrid reduziert.

2. Verfahren gemäss Anspruch 1, dadurch gekennzeichnet, dass man die Umsetzung des Cycloanhydrides der Formel II mit einem Alkohol der Formel III in Gegenwart eines tert. Amins als Katalysator, beispielsweise in Gegenwart von Triäthylamin, durchführt.

3. Verfahren gemäss Anspruch 2, dadurch gekennzeichnet, dass man den Katalysator in stöchiometrischen Mengen verwendet.

4. Verfahren gemäss einem der Ansprüche 1—3, dadurch gekennzeichnet, dass man die Reduktion eines Halbesters der Formel IV mit Lithiumborhydrid durchführt.

5. Verfahren gemäss einem der Ansprüche 1—4, dadurch gekennzeichnet, dass man sowohl die Umsetzung des Cycloanhydrids der Formel II mit einem Alkohol der Formel III als auch die Reduktion eines Halbesters der Formel IV unter Inertgas durchführt.

6. Verfahren gemäss einem der Ansprüche 1—5, dadurch gekennzeichnet, dass man die Umsetzung des Cycloanhydrids der Formel II mit einem Alkohol der Formel III bei einer Temperatur von etwa −50°C bis etwa Raumtemperatur durchführt.

7. Verfahren gemäss einem der Ansprüche 1—6, dadurch gekennzeichnet, dass man einen Alkohol der Formel III verwendet, worin $R^1$ einen Rest der Formel (d) darstellt.

8. Verfahren gemäss Anspruch 7, dadurch gekennzeichnet, dass man als Alkohol der Formel III ein [S]-1,1-Diarylpropanol oder ein [S]-1,1-Diarylpropan-1,2-diol verwendet.

9. Verfahren gemäss einem der Ansprüche 1—8, dadurch gekennzeichnet, dass man das Cycloanhydrid der Formel II mit [S]-1,1-Diphenylpropanol oder [S]-1,1-Diphenyl-1,2-propandiol in einem aromatischen Kohlenwasserstoff bei einer Temperatur von etwa −20°C bis etwa 0°C in Gegenwart von Diazabicyclooctan durchführt.

**Revendications pour les Etats contractants: BE CH DE FR GB IT LI NL**

1. Procédé de préparation de la lactone, possédant une activité optique, de formule

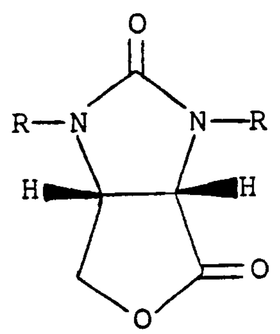

(3aS,6aR) I

dans laquelle R représente le groupe benzyle, caractérisé en ce que l'on fait réagir le cycloanhydride de formule

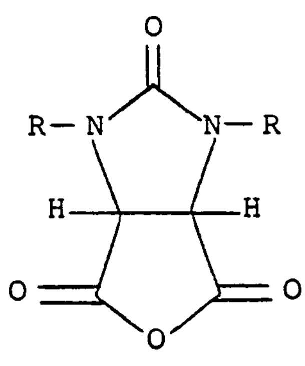

II

dans lequelles R a la signification indiquée ci-dessus, avec un alcool secondaire chiral de formule générale

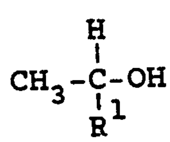

III

dans laquelle $R^1$ représente un groupe de formule

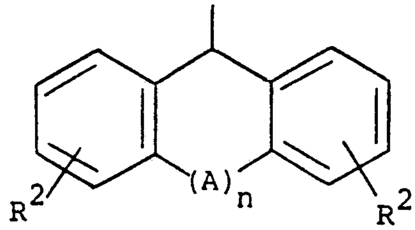

(a)

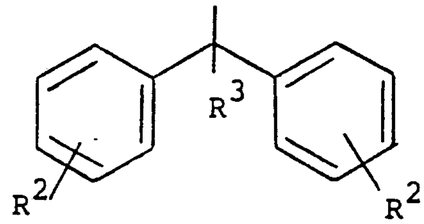

(b)

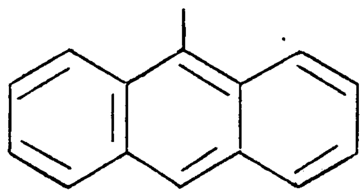

(c)

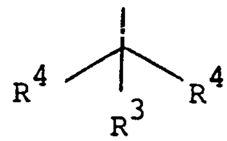

(d)

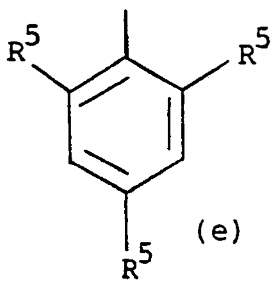

ou

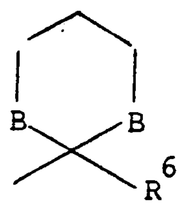

(f)

dans lesquelles $R^2$ représente l'hydrogène, un halogène, un groupe alkyle inférieur ou alcoxy inférieur, $R^3$ représente l'hydrogène ou un groupe hydroxy, ou bien encore, lorsque $R^2$, dans le groupe (b) représente l'hydrogène, un groupe alkyle inférieur, alcoxy inférieur ou phényle, $R^4$ représente un groupe cycloalkyle, un groupe phényle éventuellement substitué par le chlore ou des groupes méthyle, thiényle ou 2-furyle, $R^5$ représente l'hydrogène ou un groupe alkyle inférieur, $R^6$ représente un groupe alkyle inférieur ou phényle, A représente le soufre ou un groupe méthylène, B représente le soufre, un groupe $—SO_2—$ ou un groupe méthylène et n est égal à 1 lorsque A représente le soufre ou à 1 ou 2 lorsque A représente un groupe méthylène, ce qui donne l'hémiester de formule générale

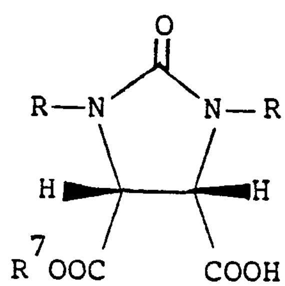

IV

dans laquelle $R^7$ représente le groupe

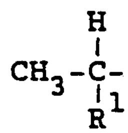

et R et $R^1$ ont les significations indiquées ci-dessus, qu'on réduit par un hydrure du bore complexe.

2. Procédé selon la revendication 1, caractérisé en ce que la réaction du cycloanhydride de formule II avec un alcool de formule III est effectuée en présence d'une amine tertiaire servant de catalyseur, par exemple en présence de triéthylamine.

3. Procédé selon la revendication 2, caractérisé en ce que l'on utilise le catalyseur en quantité stoechiométrique.

4. Procédé selon l'une des revendications 1 à 3, caractérisé en ce que la réduction d'un hémiester de formule IV est réalisée à l'aide du borohydrure de lithium.

5. Procédé selon l'une des revendications 1 à 4, caractérisé en ce que la réaction du cycloanhydride de formule II avec un alcool de formule III et la réduction d'un hémiester de formule IV sont réalisées en atmosphère de gaz inerte.

6. Procédé selon l'une des revendications 1 à 5, caractérisé en ce que la réaction du cycloanhydride de formule II avec un alcool de formule III est réalisée à une température allant d'environ −50°C jusqu'au voisinage de la température ambiante.

7. Procédé selon l'une des revendications 1 à 6, caractérisé en ce que l'on utilise un alcool de formule III dans laquelle $R^1$ représente un groupe de formule (d).

8. Procédé selon la revendication 7, caractérisé en ce que l'on utilise en tant qu'alcool de formule III un (S)-1,1-diaryl-propanol ou un (S)-1,1-diarylpropane-1,2-diol.

9. Procédé selon l'une des revendications 1 à 8, caractérisé en ce que l'on fait réagir le cycloanhydride de formule II avec le (S)-1,1-diphénylpropanol ou le (S)-1,1-diphényl-1,2-propane-diol dans un hydrocarbure aromatique à une température allant d'environ −20°C à 0°C environ, en présence du diazabicyclooctane.

10. Hémiesters, possédant un activité optique, de formule générale

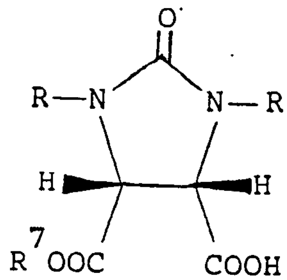

IV

dans laquelle R représente le groupe benzyle et $R^7$ le groupe

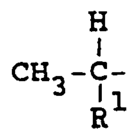

et $R^1$ représente un groupe de formule

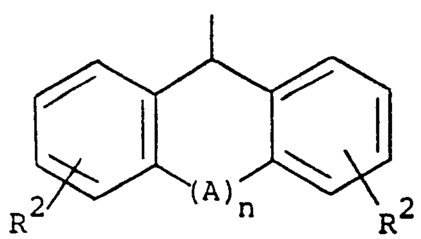

(a)

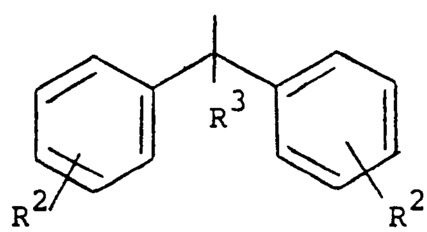

(b)

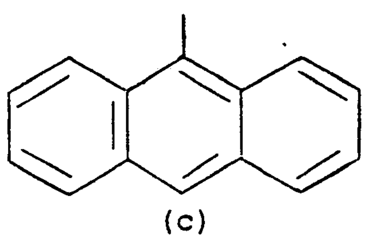

(c)

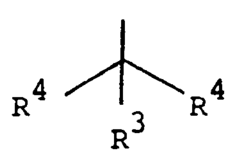

(d)

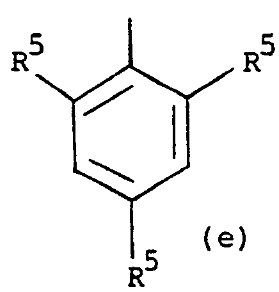

(e)

ou

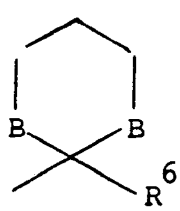

(f)

dans lesquelles $R^2$ représente l'hydrogène, un halogène, un groupe alkyle inférieur ou alcoxy inférieur, $R^3$ représente l'hydrogène ou un groupe hydroxy, ou bien encore, lorsque $R^2$, dans le groupe (b) représente l'hydrogène, un groupe alkyle inférieur, alcoxy inférieur ou phényle, $R^4$ représente un groupe cycloalkyle, un groupe phényle éventuellement substitué par le chlore ou des groupes méthyle, thiényle ou 2-furyle, $R^5$ représente l'hydrogène ou un groupe alkyle inférieur, $R^6$ représente un groupe alkyle inférieur ou phényle, A représente le soufre ou un groupe méthylène, B représente le soufre, un groupe $—SO_2—$ ou un groupe méthylène et n est égal à 1 lorsque A représente le soufre ou a 1 ou 2 lorsque A représente un groupe méthylène.

**Revendications pour l'Etat contractant: AT**

1. Procédé de préparation de la lactone, possédant une activité optique, de formule

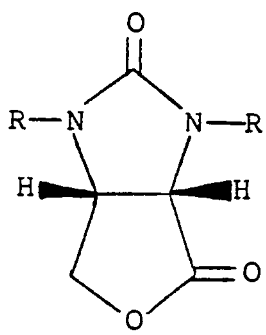

(3aS,6aR) I

dans laquelle R représente le groupe benzyle, caractérisé en ce que l'on fait réagir le cycloanhydride de formule

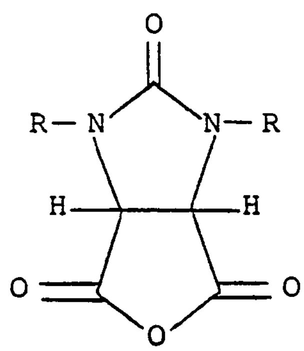

II

dans lequelles R a la signification indiquée ci-dessus, avec un alcool secondaire chiral de formule générale

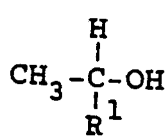

III

dans laquelle $R^1$ représente un groupe de formule

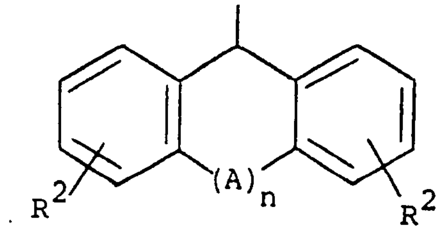

(a)

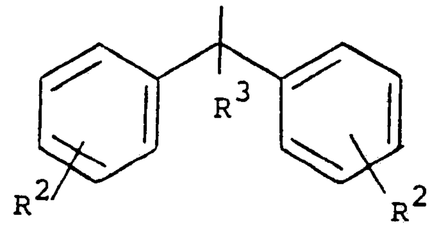

(b)

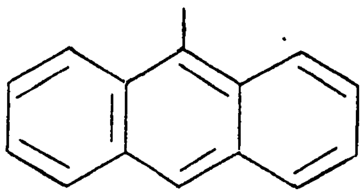

(c)

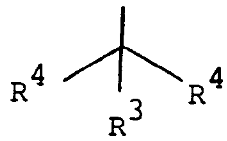

(d)

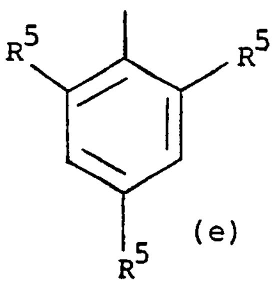

(e)

ou

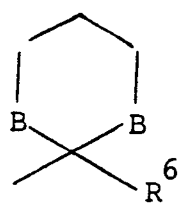

(f)

dans lesquelles $R^2$ représente l'hydrogène, un halogène, un groupe alkyle inférieur ou alcoxy inférieur, $R^3$ représente l'hydrogène ou un groupe hydroxy, ou bien encore, lorsque $R^2$, dans le groupe (b) représente l'hydrogène, un groupe alkyle inférieur, alcoxy inférieur ou phényle, $R^4$ représente un groupe cycloalkyle, un groupe phényle éventuellement substitué par le chlore ou des groupes méthyle, thiényle ou 2-furyle, $R^5$ représente l'hydrogène ou un groupe alkyle inférieur, $R^6$ représente un groupe alkyle inférieur ou phényle, A représente le soufre ou un groupe méthylène, B représente le soufre, un groupe $—SO_2—$ ou un groupe méthylène et n est égal à 1 lorsque A représente le soufre ou à 1 ou 2 lorsque A représente un groupe méthylène, ce qui donne l'hémiester de formule générale

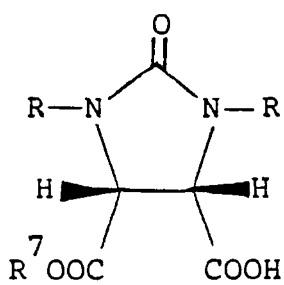

IV

dans laquelle $R^7$ représente le groupe

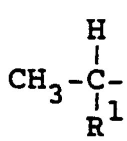

et R et $R^1$ ont les significations indiquées ci-dessus, qu'on réduit par un hydrure de bore complexe.

2. Procédé selon la revendication 1, caractérisé en ce que la réaction de cycloanhydride de formule II avec un alcool de formule III est effectuée en présence d'une amine tertiaire servant de catalyseur, par exemple en présence de triéthylamine.

3. Procédé selon la revendication 2, caractérisé en ce que l'on utilise le catalyseur en quantité stoechiométrique.

4. Procédé selon l'une des revendications 1 à 3, caractérisé en ce que la réduction d'un hémiester de formule IV est réalisée à l'aide du borohydrure de lithium.

5. Procédé selon l'une des revendications 1 à 4, caractérisé en ce que la réaction du cycloanhydride de formule II avec un alcool de formule III et la réduction d'un hémiester de formule IV sont réalisées en atmosphère de gaz inerte.

6. Procédé selon l'une des revendications 1 à 5, caractérisé en ce que la réaction du cycloanhydride de formule II avec un alcool de formule III est réalisée à une température allant d'environ −50°C jusqu'au voisinage de la température ambiante.

7. Procédé selon l'une des revendications 1 à 6, caractérisé en ce que l'on utilise un alcool de formule III dans laquelle $R^1$ représente un groupe de formule (d).

8. Procédé selon la revendication 7, caractérisé en ce que l'on utilise en tant qu'alcool de formule III un (S)-1,1-diaryl-propanol ou un (S)-1,1-diarylpropane-1,2-diol.

9. Procédé selon l'une des revendications 1 à 8, caractérisé en ce que l'on fait réagir le cycloanhydride de formule II avec le (S)-1,1-diphénylpropanol ou le (S)-1,1-diphényl-1,2-propane-diol dans un hydrocarbure aromatique à une température allant d'environ −20°C à 0°C environ, en présence du diazabicyclooctane.

**Claims for the Contracting States: BE CH DE FR GB IT LI NL**

1. A process for the manufacture of the optically active lactone of the formula

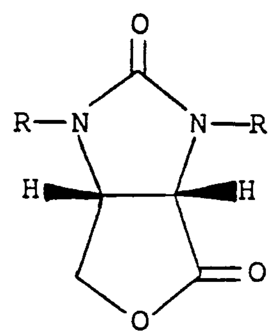

(3aS,6aR) I

wherein R represents the benzyl residue, characterized by reacting the cycloanhydride of the formula

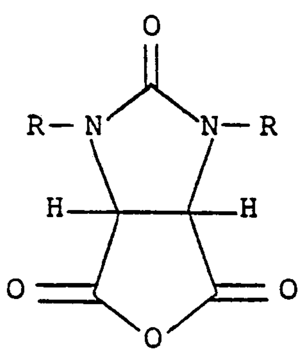

II

wherein R has the above significance, with a secondary, chiral alcohol of the general formula

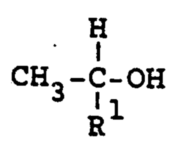

III

wherein $R^1$ represents a residue of the formula

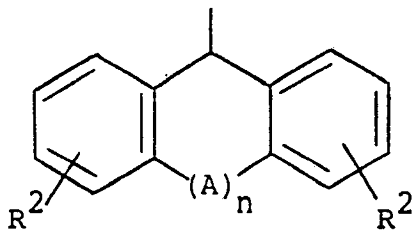

(a)

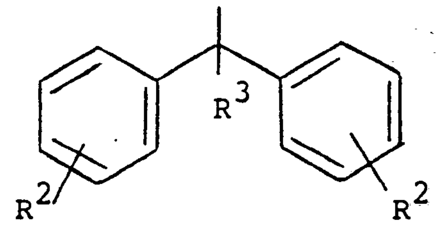

(b)

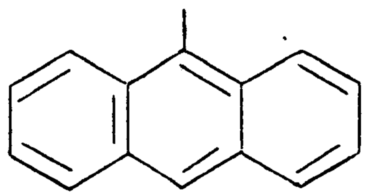

(c)

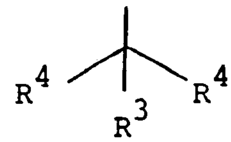

(d)

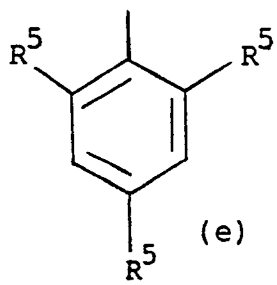

(e)

or

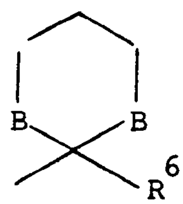

(f)

in which $R^2$ signifies hydrogen, halogen, lower alkyl or lower alkoxy, $R^3$ signifies hydrogen or hydroxy or, where $R^2$ in the residue (b) is hydrogen, $R^3$ is also lower alkyl, lower alkoxy or phenyl, $R^4$ signifies cycloalkyl, phenyl optionally substituted by chlorine or methyl, thienyl or 2-furyl, $R^5$ signifies hydrogen or lower alkyl, $R^6$ signifies lower alkyl or phenyl, A signifies sulphur or a methylene group, B signifies sulphur, $—SO_2—$ or a methylene group and n is the number 1 where A represents sulphur or is the number 1 or 2 where A signifies a methylene group, and reducing the resulting half ester of the general formula

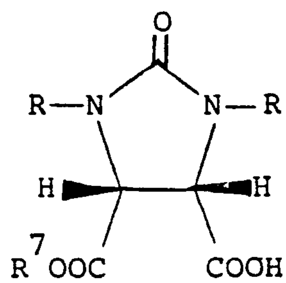

IV

wherein R′ represents the residue

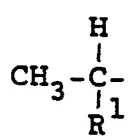

and R and $R^1$ have the above significance, with a complex borohydride.

2. A process in accordance with claim 1, characterized in that the reaction of the cycloanhydride of formula II with an alcohol of formula III is carried out in the presence of a tert. amine as a catalyst, for example in the presence of triethylamine.

3. A process in accordance with claim 2, characterized in that the catalyst is used in stoichiometric amounts.

4. A process in accordance with any one of claims 1—3, characterized in that the reduction of a half ester of formula IV is carried out with lithium borohydride.

5. A process in accordance with any one of claims 1—4, characterized in that not only the reaction of the cycloanhydride of formula II with an alcohol of formula III, but also the reduction of a half ester of formula IV is carried out under an inert gas.

6. A process in accordance with any one of claims 1—5, characterized in that the reaction of the cycloanhydride of formula II with an alcohol of formula III is carried out at a temperature of about −50°C to about room temperature.

7. A process in accordance with any one of claims 1—6, characterized in that an alcohol of formula III in which $R^1$ represents a residue of formula (d) is used.

8. A process in accordance with claim 7, characterized in that a [S]-1,1-diarylpropanol or a [S]-1,1-diarylpropane-1,2-diol is used as the alcohol of formula III.

9. A process in accordance with any one of claims 1—8, characterized in that the cycloanhydride of formula II is reacted with [S]-1,1-diphenylpropanol or [S]-1,1-diphenyl-1,2-propanediol in an aromatic hydrocarbon at a temperature of about −20°C to about 0°C in the presence of diazabicyclooctane.

10. Optically active half esters of the general formula

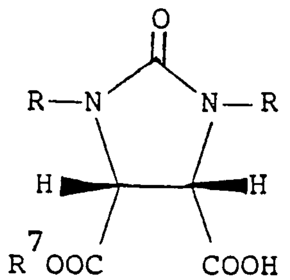

IV

wherein R represents the benzyl residue and $R^7$ represents the residue

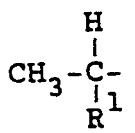

and $R^1$ represents a residue of the formula

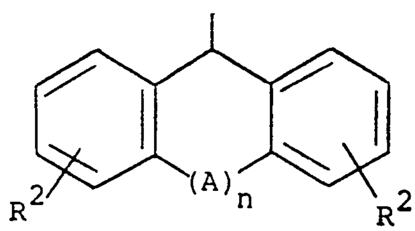

(a)

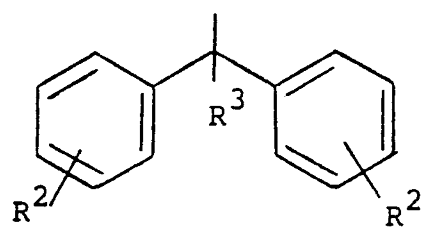

(b)

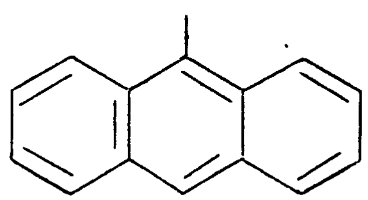

(c)

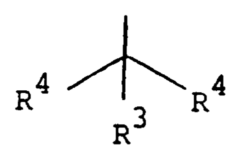

(d)

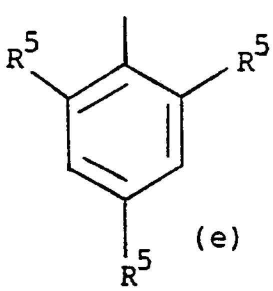

(e)

or

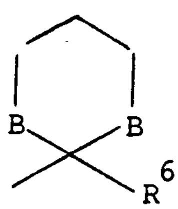

(f)

in which $R^2$ signifies hydrogen, halogen, lower alkyl or lower alkoxy, $R^3$ signifies hydrogen or hydroxy or, where $R^2$ in the residue (b) is hydrogen, $R^3$ is also lower alkyl, lower alkoxy or phenyl, $R^4$ signifies cycloalkyl, phenyl optionally substituted by chlorine or methyl, thienyl or 2-furyl, $R^5$ signifies hydrogen or lower alkyl, $R^6$ signifies lower alkyl or phenyl, A signifies sulphur or a methylene group, B signifies sulphur, $—SO_2—$ or a methylene group and n is the number 1 where A represents sulphur or is the number 1 or 2 where A signifies a methylene group.

**Claims for the Contracting State: AT**

1. A process for the manufacture of the optically active lactone of the formula

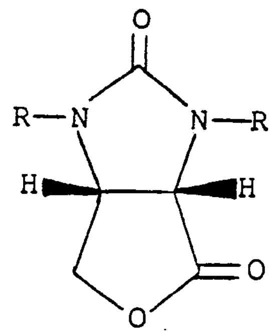

(3aS,6aR)

I

wherein R represents the benzyl residue, characterized by reacting the cycloanhydride of the formula

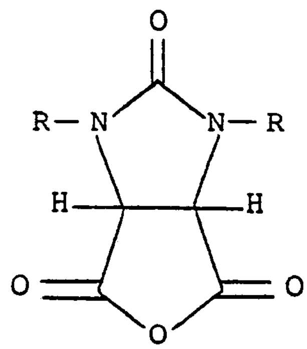

II

wherein R has the above significance, with a secondary, chiral alcohol of the general formula

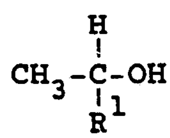

III

wherein $R^1$ represents a residue of the formula

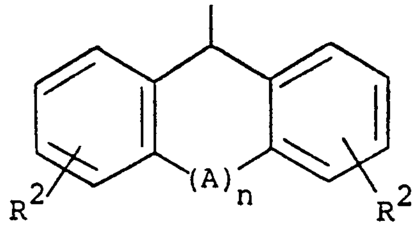

(a)

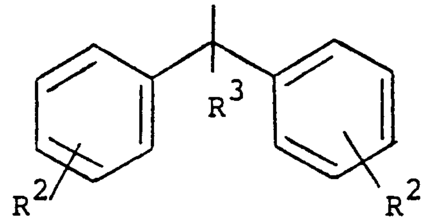

(b)

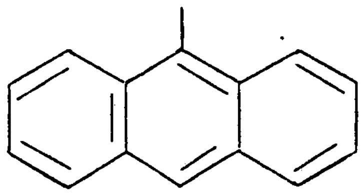

(c)

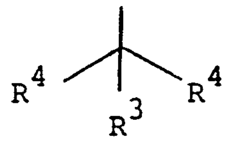

(d)

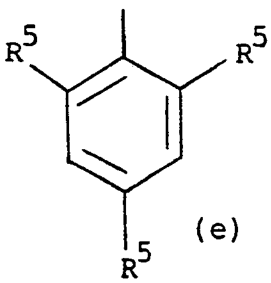

(e)

or

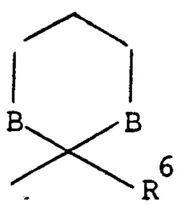

(f)

in which $R^2$ signifies hydrogen, halogen, lower alkyl or lower alkoxy, $R^3$ signifies hydrogen or hydroxy or, where $R^2$ in the residue (b) is hydrogen, $R^3$ is also lower alkyl, lower alkoxy or phenyl, $R^4$ signifies cycloalkyl, phenyl optionally substituted by chlorine or methyl, thienyl or 2-furyl, $R^5$ signifies hydrogen or lower alkyl, $R^6$ signifies lower alkyl or phenyl, A signifies sulphur or a methylene group, B signifies sulphur, $-SO_2-$ or a methylene group and n is the number 1 where A represents sulphur or is the number 1 or 2 where A signifies a methylene group and reducing the resulting half ester of the general formula

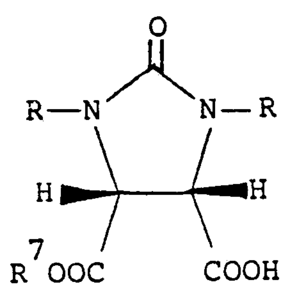

IV

wherein $R^7$ represents the residue

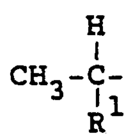

and R and $R^1$ have the above significance, with a complex borohydride.

2. A process in accordance with claim 1, characterized in that the reaction of the cycloanhydride of formula II with an alcohol of formula III is carried out in the presence of a tert. amine as a catalyst, for example in the presence of triethylamine.

3. A process in accordance with claim 2, characterized in that the catalyst is used in stoichiometric amounts.

4. A process in accordance with any one of claims 1—3, characterized in that the reduction of a half ester of formula IV is carried out with lithium borohydride.

5. A process in accordance with any one of claims 1—4, characterized in that not only the reaction of the cycloanhydride of formula II with an alcohol of formula III, but also the reduction of a half ester of formula IV is carried out under an inert gas.

6. A process in accordance with any one of claims 1—5, characterized in that the reaction of the cycloanhydride of formula II with an alcohol of formula III is carried out at a temperature of about −50°C to about room temperature.

7. A process in accordance with any one of claims 1—6, characterized in that an alcohol of formula III in which $R^1$ represents a residue of formula (d) is used.

8. A process in accordance with claim 7, characterized in that a [S]-1,1-diarylpropanol or a [S]-1,1-diarylpropane-1,2-diol is used as the alcohol of formula III.

9. A process in accordance with any one of claims 1—8, characterized in that the cycloanhydride of formula II is reacted with [S]-1,1-diphenylpropanol or [S]-1,1-diphenyl-1,2-propanediol in an aromatic hydrocarbon at a temperature of about −20°C to about 0°C in the presence of diazabicyclooctane.